# EUROPÄISCHE PATENTSCHRIFT

(11) **EP 0 975 790 B1**
(45) Veröffentlichungstag und Bekanntmachung des Hinweises auf die Patenterteilung: **29.09.2004**
(21) Anmeldenummer: 98905314.5
(22) Anmeldetag: 20.01.1998
(51) Int. Cl.: C12P 19/04, A61K 39/02, A61K 39/385, A61K 47/36

(54) **NEUES VERFAHREN ZUR ISOLIERUNG VON POLYSACCHARIDEN**
NEW METHOD FOR ISOLATING POLYSACCHARIDES
NOUVEAU PROCEDE POUR ISOLER DES POLYSACCHARIDES

(30) Priorität: 24.01.1997 EP 97101143
(43) Veröffentlichungstag der Anmeldung: 02.02.2000
(73) Patentinhaber: Schweiz. Serum-&Impfinstitut Bern, 3001 Bern (CH)
(72) Erfinder: HASLER, Thomas, CH-3007 Bern (CH); FÜRER, Emil, CH-3074 Muri (CH)
(74) Vertreter: VOSSIUS & PARTNER
(86) Internationale Anmeldenummer: PCT/EP1998/000268
(87) Internationale Veröffentlichungsnummer: WO 1998/032873

(56) Entgegenhaltungen:
- EP-A- 0 407 037
- E. C. GOTSCHLICH ET AL.: "Human Immunity to the Meningococcus." J. EXP. MED., Bd. 129, Nr. 4, 1969, Seiten 1349-1365, XP002063730 in der Anmeldung erwähnt

## Beschreibung

Die Erfindung betrifft ein Verfahren zur Isolierung von Polysacchariden, insbesondere zur Abtrennung von Endotoxinen von Kapsel-Polysacchariden gram-negativer Bakterien. Die Beschreibung der Erfindung erläutert, daß die mit dem erfindungsgemäßen Verfahren isolierten Polysaccharide vorzugsweise zur Herstellung von Polysaccharidimpfstoffen verwendet werden können. Die Erfindungsbeschreibung erläutert ferner impfstoffe, die mit dem erfindungsgemäßen Verfahren isolierte Polysaccharide enthalten.

Für die Herstellung von Impfstoffen, insbesondere Polysaccharidimpfstoffen aus bakteriellen Polysacchariden ist die Entfernung von Endotoxinen ein kritischer und entscheidender Schritt im Verlaufe der Reinigung der Polysaccharide. Das im Stand der Technik am häufigsten verwendete Verfahren zur Abtrennung von Endotoxinen von bakteriellen Polysacchariden beruht auf einer Phenolextraktion, die gegebenenfalls mehrfach wiederholt werden muß, bis der Endotoxingehalt den von den Gesundheitsbehörden erhobenen Anforderungen entspricht. Dieses Verfahren ist aufwendig und zeitraubend. Darüber hinaus ist die Arbeit mit Phenol unangenehm und verursacht unerwünschten toxischen Abfall. Außerdem sind die mit diesem aus dem Stand der Technik bekannten Verfahren erzielbaren Ausbeuten an Polysacchariden häufig unbefriedigend. Andere Verfahren aus dem Stand der Technik zur Isolierung von bakteriellen Polysacchariden beruhen auf der Verwendung von Affinitätsäulen. Häufig sind diese für die Gesundheit bedenklich (z.B. die Verwendung von Polymyxin B-haltigem Säulenmaterial). Darüber hinaus haben viele Säulenmaterialien nur beschränkte Kapazitäten, was für die Gewinnung technisch verwertbarer Ausbeuten an Polysacchariden relativ große und damit teure Säulen bedingt (vgl. z.B. US-A 5,045,456, US-A 5,039,610 und US-A 5,034,519).

Des weiteren sind im Stand der Technik Verfahren zur Isolierung von bakteriellen Polysacchariden bekannt, welche komplexe Teilschritte umfassen. Die europäische Patentanmeldung EP-A-0407037 (Rienstra et al.) beschreibt ein komplexes Verfahren, wobei eine Polysaccharidfraktion, die durch entsprechende Kultivierung gram-negativer Bakterien und anschließender Aufarbeitung der Kultur erhalten worden war, zunächst einer aufwendigen Phenolextraktion unterworfen wird. Die hochmolekularen Substanzen werden mit Alkohol und Calciumchlorid ausgefällt, der Lösung wird erneut Calciumchlorid zugesetzt und das Endotoxin wird durch selektive Alkoholfraktionierung und anschließender Zugabe eines Trägermaterials, eines Detergens und eines Chelatbildners vom Polysaccharid abgetrennt. Das Trägermaterial wird aus der Lösung entfernt, das Filtrat über Hohlfasermembranen von Detergens von dem Chelatbildner befreit, der Rückstand mit Calciumchlorid versetzt und das Polysaccharid schließlich mit Alkohol aus der Lösung ausgefällt. EP-A-0407037 offenbart zwar das Entfernen von Lipopolysacchariden durch selektive Alkoholfraktionierung, allerdings nur in Kombination mit der Extraktion über ein Trägermaterial. EP-A-0407037 beschreibt daher ein Verfahren, bei dem die bakterielle Polysaccharidfraktion weiteren wesentlichen Verfahrenschritten unterworfen wird, welche die Aufreinigung der Polysaccharide erst möglich machen. Des weiteren kann das Verfahren in EP-A-0407037 nur unter Anwendung von Extraktionsschritten mit toxischen Lösungsmitteln durchgeführt werden.

Der Erfindung lag somit die Aufgabe zugrunde, ein vereinfachtes, wirtschaftlich sinnvolles und für die Gesundheit weniger belastendes Verfahren zur Isolierung von Polysacchariden bereitzustellen. Die Lösung dieser Aufgabe wird durch die in den Ansprüchen gekennzeichneten Ausführungsformen erreicht.

Die Erfindung betrifft somit ein Verfahren zur Isolierung von Polysacchariden, wobei man folgende Schritte durchführt:
(a) Mischen einer bakteriellen Polysaccharidfraktion mit einer Detergenslösung;
(b) Alkoholzugabe zu einer Endkonzentration, die unter der Konzentration liegt, bei der das Polysaccharid ausfällt;
(c) Mischen der Lösung;
(d) Filtrieren der Lösung;
(e) Abtrennung des Polysaccharids von Detergens und Alkohol.

Bakterielle Polysaccharidfraktionen, die im erfindungsgemäßen Verfahren eingesetzt werden können, sind durch im Stand der Technik bekannte Verfahren herstellbar; vgl. z.B. Gotschlich et al., J. Exp. Med. 129 (1969), 1349-1365 sowie Schneerson et al., J. Exp. Med. 152 (1980) 361-376. Die Alkoholkonzentration, bei der das Polysaccharid in Gegenwart der Detergenslösung ausfällt, ist für den Fachmann auf konventionelle Weise bestimmbar. Beispielsweise kann diese Konzentration durch einfache Testreihen ermittelt werden.

Das Umsetzen der Lösung, d.h. das Ausfällen des Endotoxins aus der Polysaccharidlösung erfolgt üblicherweise 1 Minute .bis 1 Stunde, kann aber auch mehrere Stunden erfolgen. Das erfindungsgemäße Verfahren ist im Gegensatz zu den im Stand der Technik bekannten Verfahren einfach, schnell, billig und verursacht weniger toxischen Abfall. Darüber hinaus sind die Ausbeuten an Polysaccharid deutlich höher. Das erfindungsgemäße Verfahren beruht auf einer selektiven Alkoholfällung in Gegenwart von mindestens einem Detergens, welches nicht kovalente Interaktionen zwischen Polysacchariden, Lipopolysacchariden und Proteinen aufhebt.

In einer bevorzugten Ausführungsform des erfindungsgemäßen Verfahrens ist der zuzugebende Alkohol Ethanol.

In einer weiteren bevorzugten Ausführungsform des erfindungsgemäßen Verfahrens erfolgt die Abtrennung des Polysaccharids von Detergens und Alkohol durch Fällung des Polysaccharids durch weitere Alkoholzugabe.
Diese Ausführungsform des Verfahrens ist besonders vorteilhaft, da die Polysaccharidfällung und damit die Abtrennung von Detergentien und Alkohol durch einfache weitere Zugabe des Alkohols erreicht werden kann. In einer anderen Ausführungsform kann die Fällung des Polysaccharids auch durch Zugabe eines Alkohols erreicht werden, der sich von dem unterscheidet, der in Schritt (b) verwendet wird.

Eine weitere bevorzugte Ausführungsform der Erfindung betrifft ein Verfahren, wobei die Polysaccharide aus gram-negativen Bakterien stammen. In einer besonders bevorzugten Ausführungsform sind die gram-negativen Bakterien Bakterien der Gattung Haemophilus, Neisseria, Klebsiella oder Escherichia und insbesondere der Art Haemophilus influenzae (Typ b), Neisseria meningitidis, Klebsiella pmeumoniae oder Escherichia coli. Bei den hier in Rede stehenden Polysacchariden handelt es sich um Kapsel-Polysaccharide.

Die Isolierung von Polysacchariden aus Bakterien dieser Gattungen bzw. Arten ist deshalb besonders bevorzugt, da sich diese Polysaccharide zur Impfung gegen folgende Krankheiten eignen: Meningitis, Epiglottitis, Otitis media, Pneumonie, Arthritis, Sepsis, nosokomiale Infektionen, Harnwegsinfektionen und Gastroenteritis.

In einer weiteren bevorzugten Ausführungsform des erfindungsgemäßen Verfahrens ist das Detergens ein anionisches Tensid. Besonders bevorzugt ist ein Verfahren, wobei das anionische Tensid ein Alkylsulfat, beispielsweise Natriumdodecylsulfat (SDS) ist.
Der Vorteil des Einsatzes von SDS im erfindungsgemäßen Verfahren liegt u.a. darin, daß SDS von einer Vielzahl von Herstellern bezogen werden kann und darüber einen günstigen Verkaufspreis aufweist.

In einer weiteren besonders bevorzugten Ausführungsform des erfindungsgemäßen Verfahrens ist die Tensidkonzentration in der der Polysaccharidfraktion in dem oben genannten Schritt (a) zugesetzten Lösung höchstens 20 % (Gew./Gew.). Wie bereits vorstehend erwähnt, ist das Tensid vorzugsweise ein Alkylsulfat und beispielsweise SDS.

Erfindungsgemäß besonders bevorzugt ist ein Verfahren, wobei die Tensidkonzentration in der Polysaccharidlösung, beispielsweise die SDS-Konzentration, 0,1 % bis 4 % (Endkonzentration, Gew./Gew.) beträgt.

In einer weiteren bevorzugten Ausführungsform des erfindungsgemäßen Verfahrens wird der Alkohol in Schritt (b) zu einer Endkonzentration zu der Lösung gegeben, die etwa 10 % unter der Konzentration liegt, bei der das Polysaccharid ausfällt.
Es hat sich durch empirische Testreihen herausgestellt, daß die Zugabe des Alkohols im Schritt (b) zu dieser Endkonzentration besonders vorteilhaft ist, weil der Verlust an Polysaccharid bei dieser Konzentration gering ist und Endotoxin trotzdem effizient ausgefällt wird.

In einer weiteren bevorzugten Ausführungsform des erfindungsgemäßen Verfahrens ist die Ausgangskonzentration an Polysacchariden in der Polysaccharidfraktion größer als 10 mg/ml.
Während das erfindungsgemäße Verfahren auch mit geringeren Ausgangskonzentrationen an Polysacchariden in der Polysaccharidfraktion durchgeführt werden kann, sollte die vorstehend genannte Konzentration als Mindestkonzentration besonders aus wirtschaftlichen Gesichtspunkten im erfindungsgemäßen Verfahren eingesetzt werden.

Eine weitere bevorzugte Ausführungsform der Erfindung betrifft ein Verfahren, wobei die Filtration durch einen Polymerfilter erfolgt.

In einer anderen bevorzugten Ausführungsform des erfindungsgemäßen Verfahrens wird für die Filtration ein Tiefenfilter eingesetzt.
Der Begriff "Tiefenfilter" bedeutet im Zusammenhang mit der vorliegenden Erfindung einen Filter, der im Gegensatz zu einem Membranfilter (2-dimensional) eine 3-dimensionale Struktur (Tiefe) besitzt. Dieser Aufbau hat zur Folge, daß ein Tiefenfilter eine hohe Partikelrückhaltekapazität besitzt und entsprechend nicht so schnell verstopft.
Der Einsatz von Polymer- bzw. Tiefenfiltern hat sich erfindungsgemäß besonders bewährt. Dabei ist zu berücksichtigen, daß ein Polymerfilter auch Tiefenfilter und umgekehrt ein Tiefenfilter auch ein Polymerfilter sein kann, daß diese Bedingung jedoch nicht zwingend ist.
Die Isolierung der Polysaccharide nach dem erfindungsgemäßen Verfahren stellt sich als besonders effizient dar, wenn zur Filtration Tiefenfilter eingesetzt werden.

In einer besonders bevorzugten Ausführungsform des erfindungsgemäßen Verfahrens ist der Polymerfilter und/oder der Tiefenfilter ein Polypropylenfilter.

Die Beschreibung der Erfindung erläutert ferner einen Polysaccharidimpfstoff, der dadurch gekennzeichnet ist, daß er ein Polysaccharid enthält, das nach dem erfindungsgemäßen Verfahren isoliert wurde. Gegebenenfalls enthält dieser Polysaccharidimpfstoff ferner einen pharmazeutisch verträglichen Träger. Beispiele für derartige Träger sind Tetanustoxoid, Diphtherietoxoid, Pseudomonas Exotoxin A und Choleratoxin.
Der Polysaccharidimpfstoff ist wie den vorstehenden Ausführungen entnommen werden kann, besonders einfach und billig herstellbar. Seine Herstellung ist darüber hinaus für das Laborpersonal aus gesundheitlicher Sicht besonders unbedenklich. Ein Beispiel für den Impfstoff ist ein auf Meningokokkenpolysaccharid basierender Impfstoff. Diese wie auch die nachfolgend genannten Beispiele des Impfstoffs werden parenteral verabreicht, wobei die Verabreichung ein bis mehrere Male und vorzugsweise (wo nicht anders angezeigt) mehrere Male erfolgt. Üblicherweise wird die Verabreichung intramuskular oder subkutan verabreicht, wobei pro Dosis 1-50 µg Polysaccharid eingesetzt werden.

Der Polysaccharidimpfstoff eignet sich insbesondere zur Impfung gegen Meningitis, Epiglottitis, Otitis media, Pneumonie, Arthritis, Sepsis, nosokomiale Infektionen, Harnwegsinfektionen oder Gastroenteritis. Darüber hinaus kann der Polysaccharidimpfstoff jedoch auch für die Impfung gegen andere Krankheiten eingesetzt werden, die durch Kapselpolysaccharide tragende gram-negative Bakterien verursacht werden.

Außerdem erläutert die Beschreibung der Erfindung ein Konjugat, das aus einem nach dem erfindungsgemäßen Verfahren isolierten Polysaccharid und einem chemisch damit verknüpften pharmazeutisch verträglichen Protein besteht. Beispiele für derartige Proteine sind Tetanustoxoid, Diphtherietoxoid, Pseudomonas Exotoxin A und Choleratoxin. Dosen umfassen 1-20 µg an Konjugat.

Darüber hinaus erläutert die Beschreibung der Erfindung einen Konjugatimpfstoff, der ein nach dem erfindungsgemäßen Verfahren isoliertes Polysaccharid und chemisch damit verknüpft ein pharmazeutisch verträgliches Protein enthält.

Der Konjugatimpfstoff wird bevorzugt für die Immunisierung bzw. Prophylaxe gegen die vorgenannten Krankheiten eingesetzt.

Insbesondere erwähnt ist dabei, daß die Immunisierung bei Kleinkindern durchgeführt wird.

Die Beschreibung der Erfindung erläutert des weiteren einen Kombinationsimpfstoff, der ein nach dem erfindungsgemäßen Verfahren isoliertes Polysaccharid oder ein Konjugat sowie eine weitere immunogene Komponente enthält, wobei die zusätzliche immunogene Komponente vorzugsweise eine Immunantwort gegen ein Pathogen induziert, das ein anderes Pathogen als das ist, aus dem das Polysaccharid stammt. Beispiele für einen Kombinationsimpfstoff ist ein Haemophilus influenzae-Impfstoff, in dem das entsprechende Polysaccharid mit Tetanustoxoid konjugiert ist. Zusätzlich können in diesem Impfstoff z.B. Pertussis-, Diphtherie-, Tetanus- und Hepatitis B-Komponenten formuliert werden. Dosen umfassen 1-20 µg an Polysaccharid im Kombinationsimpfstoff, insbesondere 1-10 µg, beispielsweise beim vorstehend beschriebenen Haemophilus influenzae Kombinationsimpfstoff an Haemophilis influenzae-Polysaccharid. Dosen für Diphtheriekomponenten sind in diesem Impfstoff 15-25 Lf (Limit of flocculation), für Tetanuskomponenten 5-10 Lf und für Pertussiskomponenten mehr als 4 IE (Internationale Einheiten). Der Fachmann kann Dosen/Konzentrationen weiterer Komponenten im Kombinationsimpfstoff nach Standardverfahren/Standardvorschriften bestimmen. Der Kombinationsimpfstoff wird nur einmal verabreicht.

Die Beschreibung erläutert, daß die weitere immunogene Komponente ein Diphterie-, Tetanus-, Pertussis-, Hepatitis B- oder Poliomyelitis-Antigen.

Schließlich erläutert die Beschreibung der Erfindung die Verwendung eines Polysaccharids, das nach dem erfindungsgemäßen Verfahren isoliert wurde, als Zwischenprodukt für die Herstellung eines Konjugat- oder Kombinationsimpfstoffes. Das Zwischenprodukt wird dabei mit einem pharmazeutisch verträglichen Protein chemisch zum Konjugat verknüpft. Dementsprechend erläutert die Beschreibung der Erfindung insbesondere eine Verwendung, wobei der Konjugat- oder Kombinationsimpfstoff als Wirkkomponente ein Konjugat enthält, das aus einem nach dem erfindungsgemäßen Verfahren isolierten Polysaccharid und einem chemisch damit verknüpften pharmazeutisch verträglichen Protein besteht.

Die Beispiele erläutern die Erfindung.

### Beispiel 1

### Isolierung eines Haemophilus influenzae Typ b Kapsel-Polysaccharids

Eine nach konventionellen Verfahren aufgearbeitete Kapsel-Polysaccharidfraktion (PRP, Polyribosylribitolphosphat) aus Haemophilus influenzae Typ b wird in einer Konzentration von > 10 mg/ml mit einer 4%-igen SDS-Lösung gemischt. Daraufhin wird Ethanol zu einer Endkonzentration zugegeben, die etwa 10 % unter der Konzentration liegt, bei der das Polysaccharid auszufallen beginnt. Die Lösung wird etwa 20 Minuten lang gemischt, wobei auch Zeiträume von 1 Minute bis mehrere Stunden geeignet erscheinen, worauf sich eine leichte Trübung einstellt. Anschließend wird durch einen Polypropylen-Tiefenfilter filtriert. Die Endotoxine werden durch diesen Filtrationsschritt abgetrennt und verbleiben im Filter. Vermutlich sind sowohl Filtrations- als auch Adsorptionseffekte für die Abtrennung der Endotoxine verantwortlich. Das filtrierte Polysaccharid wird anschließend durch weitere Ethanolzugabe gefällt, wobei das SDS in Lösung verbleibt. Das gefällte Polysaccharid kann durch weitere Ethanolfällungen von bleibenden SDS-Verunreinigungen abgetrennt werden. Eine weitere Aufarbeitung des Polysaccharides sowie die Konfektionierung als Impfstoff, wobei das Polysaccharid vorzugsweise chemisch mit einem geeigneten Trägerprotein verknüpft wird, erfolgt nach im Stand der Technik bekannten, üblichen Verfahren. In der genannten bevorzugten Ausführungsform ist das Polysaccharid auch ein Zwischenprodukt für einen Konjugatimpfstoff.

### Beispiel 2

### Isolierung von Neisseria meningitidis Typ (A) und (C) Kapsel-Polysacchariden

Neisseria meningitidis Typ (A) und (C) Kapsel-Polysaccharide wurden den gleichen Verfahrensschritten, wie in Beispiel 1 beschrieben, unterworfen.

Die Ausbeuten an mit den in den Beispielen 1 und 2 beschriebenen Verfahren erhaltenen Polysacchariden sind in Tabellen I und II dargestellt. Es zeigt sich, daß die Ausbeute an Haemophilus influenzae Typ (b) Kapsel-Polysaccharid, die mit dem erfindungsgemäßen Verfahren erhältlich ist, wesentlich höher ist als Polysaccharid, das mit im Stand der Technik bekannten Verfahren (Phenolextraktion) erhalten werden kann.

**Tabelle I**

| Isolierung von H. influenza Typ b Kapselpolysaccharid (PRP) | | | | | |
|---|---|---|---|---|---|
| PRP-Chargennummer¹ | Verfahren | PRP-Menge (g) | Endotoxin | | PRP-Ausbeute (%) |
| | | | vorher | nachher | |
| | | | (EU/µg/PRP) | | |
| 27 | 5 x Phenol | 8,3 | 475 | 26 | 67 |
| 627095 | EtOH/SDS | 1,9 | 72,5 | 0,11 | >95 |
| 611496 | EtOH/SDS | 75 | 55 | <0,05 | >95 |

| | | | | | |
|---|---|---|---|---|---|
| ¹ Bei den Chargennummern handelt es sich um interne Nummern des Anmelders, CH-Serum. Die Chargen wurden nach üblichen Verfahren hergestellt. | | | | | |

**Tabelle II**

| Isolierung von N. meningitidis Gruppe C Kapselpolysaccharid (GCMP) | | | | | |
|---|---|---|---|---|---|
| GCMP-Chargennummer¹ | Verfahren | GCMP-Menge (g) | Endotoxin | | GCMP-Ausbeute (%) |
| | | | vorher | nachher | |
| | | | (EU/µg/GCMP) | | |
| 150396 | EtOH/SDS | 7,3 | 46,8 | 7,7 | 92 |
| 905096 | EtOH/SDS | 7,5 | 258 | 1,1 | 77 |
| 906096 | EtOH/SDS | 7,8 | 85 | 0,1 | 67 |

## Patentansprüche

1. Verfahren zur Isolierung von Polysacchariden, wobei man folgende Schritte durchführt:
(a) Mischen einer bakteriellen Polysaccharidfraktion mit einer Detergenslösung;
(b) Alkoholzugabe zu einer Endkonzentration, die unter der Konzentration liegt, bei der das Polysaccharid ausfällt;
(c) Mischen der Lösung;
(d) Filtrieren der Lösung durch einen Tiefenfilter;
(e) Abtrennung des Polysaccharids von Detergens und Alkohol.

2. Verfahren nach Anspruch 1, wobei der Alkohol Ethanol ist.

3. Verfahren nach Anspruch 1 oder 2, wobei die Abtrennung des Poiysaccharids durch Fällung des Polysaccharids durch weitere Alkoholzugabe erfolgt.

4. Verfahren nach einem der Ansprüche 1 bis 3, wobei die Polysaccharide aus gram-negativen Bakterien stammen.

5. Verfahren nach Anspruch 4, wobei die gram-negativen Bakterien Bakterien der Gattung Haemophilus, Neisseria, Klebsiella oder Escherichia und insbesondere der Art Haemophilus influenzae (Typ b), Klebsiella pneumoniae, Neisseria meningitidis oder Escherichia coli sind.

6. Verfahren nach einem der Ansprüche 1 bis 5, wobei das Detergens ein anionisches Tensid ist.

7. Verfahren nach Anspruch 6, wobei das anionische Tensid ein Alkylsulfat. beispielsweise Natriumdodecylsulfat (SDS) ist.

8. Verfahren nach Anspruch 6 oder 7, wobei die Tensidkonzentration in der der Polysaccharidfraktion in Schritt (a) zugesetzten Lösung höchstens 20 % (Gew./Gew.) beträgt.

9. Verfahren nach Anspruch 8, wobei die Tensidkonzentration in der Polysaccharidlösung 0,1 % bis 4 % (Endkonzentration, Gew./Gew.) beträgt.

10. Verfahren nach einem der Ansprüche 1 bis 9, wobei der Alkohol in Schritt (b) zu einer Endkonzentration zu der Lösung gegeben wird, die etwa 10 % unter. der Konzentration liegt, bei der das Polysaccharid ausfällt.

11. Verfahren nach einem der Ansprüche 1 bis 10, wobei die Ausgangskonzentration an Polysacchariden in der Polysaccharidfraktion größer als 10 mg/ml ist.

12. Verfahren nach einem der Ansprüche 1 bis 11, wobei die Filtration durch einen Palymerfilter erfolgt.

13. Verfahren nach einem der Ansprüche 1 bis 12, wobei der Polymerfilter und/oder der Tiefenfilter ein Polypropylenfilter ist.

## Claims

1. A method for the isolation of polysaccharides wherein the following steps are carried out:
(a) mixing of a bacterial polysaccharide fraction with a detergent solution;
(b) addition of alcohol to a final concentration which is below the concentration at which the polysaccharide precipitates;
(c) mixing the solution;
(d) filtering the solution by means of a deep bed filter;
(e) separation of the polysaccharide from detergent and alcohol.

2. The method of claim 1 wherein the alcohol is ethanol.

3. The method of claim 1 or 2 wherein the separation of the polysaccharide is carried out by the precipitation of the polysaccharide by adding more alcohol.

4. The method of any one of claims 1 to 3 wherein the polysaccharides are derived from gram-negative bacteria.

5. The method of claim 4 wherein the gram-negative bacteria are bacteria of the genus Haemophilus, Neisseria, Klebsiella or Escherichia and in particular of the species Haemophilus influenzae (type b), Klebsiella pneumoniae, Neisseria meningitidis or Escherichia coli.

6. The method of any one of claims 1 to 5 wherein the detergent is an anionic surfactant.

7. The method of claim 6 wherein the anionic surfactant is an alkyl sulfate, for example, sodium dodecyl sulfate (SDS).

8. The method of claim 6 or 7 wherein the surfactant concentration in the solution added to the polysaccharide fraction in step (a) is at the most 20% (w/w).

9. The method of claim 8 wherein the surfactant concentration in the polysaccharide solution is 0.1% to 4% (final concentration, w/w).

10. The method of any one of claims 1 to 9 wherein in step (b) the alcohol is added to the solution to a final concentration which is approximately 10% below the concentration at which the polysaccharide precipitates.

11. The method of any one of claims 1 to 10 wherein the initial concentration of polysaccharides in the polysaccharide fraction is greater than 10 mg/ml.

12. The method of any one of claims 1 to 11 wherein the filtration is carried out by means of a polymer filter.

13. The method of any one of claims 1 to 12 wherein the polymer filter and/or the deep bed filter is a polypropylene filter.

## Revendications

1. Procédé pour isoler des polysaccharides, dans lequel on réalise les étapes suivantes :
(a) on mélange une fraction de polysaccharide bactérien avec une solution détergente ;
(b) on ajoute un alcool à une concentration finale qui est inférieure à la concentration à laquelle le polysaccharide précipite ;
(c) on mélange la solution ;
(d) on filtre la solution au moyen d'un filtre en profondeur ;
(e) on sépare le polysaccharide du détergent et de l'alcool.

2. Procédé selon la revendication 1, dans lequel l'alcool est l'éthanol.

3. Procédé selon la revendication 1 ou 2, dans lequel la séparation du polysaccharide est réalisée par la précipitation du polysaccharide en ajoutant d'avantage d'alcool.

4. Procédé selon l'une quelconque des revendications 1 à 3, dans lequel les polysaccharides sont dérivés de bactéries gram-négatives.

5. Procédé selon la revendication 4, dans lequel les bactéries gram-négatives sont des bactéries du genre Haemophilus, Neisseria, Klebsiella ou Escherichia, et en particulier des espèces Haemophilus influenzae (type b), Klebsiella pneumoniae, Neisseria meningitidis ou Escherichia coli.

6. Procédé selon l'une quelconque des revendications 1 à 5, dans lequel le détergent est un surfactant anionique.

7. Procédé selon la revendication 6, dans lequel le surfactant anionique est un sulfate d'alkyle, par exemple le sulfate de dodécyle sodium (SDS).

8. Procédé selon la revendication 6 ou 7, dans lequel la concentration en surfactant dans la solution ajoutée à la fraction de polysaccharide dans l'étape (a) est au plus 20% (w/w).

9. Procédé selon la revendication 8, dans lequel la concentration en surfactant dans la solution de polysaccharide est 0,1% à 4% (concentration finale, w/w).

10. Procédé selon l'une quelconque des revendications 1 à 9, dans lequel, dans l'étape (b), l'alcool est ajouté à la solution à une concentration finale qui est environ 10% inférieure à la concentration à laquelle le polysaccharide précipite.

11. Procédé selon l'une quelconque des revendications 1 à 10, dans lequel la concentration initiale en polysaccharides dans la fraction de polysaccharide est supérieure à 10 mg/ml.

12. Procédé selon l'une quelconque des revendications 1 à 11, dans lequel la filtration est réalisée au moyen d'un filtre polymère.

13. Procédé selon l'une quelconque des revendications 1 à 12, dans lequel le filtre polymère et/ou le filtre en profondeur est un filtre en polypropylène.
